(12) **EUROPEAN PATENT APPLICATION**

(21) Application number: **84306474.2**

(22) Date of filing: **21.09.84**

(51) Int. Cl.⁴: **C 12 N 9/30**

(30) Priority: **26.09.83 DK 4382/83**

(43) Date of publication of application: **24.04.85**
**Bulletin 85/17**

(84) Designated Contracting States: **AT BE CH DE FR GB IT LI LU NL SE**

(71) Applicant: **NOVO INDUSTRI A/S, Novo Allé, DK-2880 Bagsvaerd (DK)**

(72) Inventor: **Heldt-Hansen, Hans Peter, Jaegersborg Alle 27A, 3 right, DK-2920 Charlottenlund (DK)**
Inventor: **Aunstrup, Knud, Soeparken 7, DK-3480 Fredensborg (DK)**

(74) Representative: **Brown, John David et al, FORRESTER & BOEHMERT Widenmayerstrasse 4/I, D-8000 München 22 (DE)**

(54) Acid-stable alpha-amylase composition, preparation and use thereof.

(57) A novel acid-stable alpha-amylase enzyme preparation comprising an Aspergillus derived alpha-amylase culture medium concentrate without amyloglucosidase and transferase activities. The alpha-amylase concentrate is produced by cultivating an Aspergillus niger strain mutated to block its capability to produce amyloglucosidase and transferase whilst retaining its ability to produce alpha-amylase.

EP 0 138 428 A2

Acid-stable alpha-amylase  composition, preparation and use thereof.


This invention concerns an acid-stable alpha-amylase enzyme preparation having neither amylo-glucosidase nor transferase activities.


## BACKGROUND OF THE INVENTION

It is known that fungi of the genus <u>Aspergillus</u> produce an acid-stable and an acid-unstable alpha-amylase both without maltase activity. In addition, amyloglucosidase and transferase(transglucosidase) enzyme activities are produced.

An acid-stable alpha-amylase is known from Canadian patent No. 663,274 and several articles in Agr.Biol.Chem. (Y. Minoda et al.,  Agr.Biol.Chem. Vol. 27, No. 11, p. 806 - 811, 1963, Vol. 32, No. 1, p. 104 - 109, 1968 and Vol. 32, No. 1, p. 110 - 113, 1968).

The acid-stable alpha-amylase is produced by cultivating different <u>Aspergillus</u> species, e.g. <u>A. niger</u>, <u>A. usamii</u> and <u>A.awamori</u> under acid conditions at first (pH 2.5 - 4.5) and then under slight acid conditions (pH about 5.5). In addition to the acid-stable alpha-amylase, also an acid-unstable-alpha-amylase (neutral amylase), and enzymes with glucoamylase and transferase activities are produced by the microorganism and appear in the culture medium.

0138428

According to the prior art cited above, glucoamylase and transferase activities may be removed by purification of the enzyme product, e.g. fractional precipitation with ammonium sulfate, rivanol and acetone whereby the acid-stable alpha-amylase is obtained in crystalline form.

However, as is well known in the enzyme arts crystalline enzymes are not particularly stable and otherwise are not well suited to be used by industry. Moreover, the cost of carrying out the purification and the low yields obtained vis a vis the enzyme levels present in the culture broth preclude a pricing structure for crystalline form enzymes acceptable to industry.

It is an object of the present invention to provide an  acid-stable alpha-amylase enzyme product with neither amyloglucosidase nor transferase activities which product can be obtained directly from the culture medium, thereby avoiding the above mentioned purification steps.

It is another object of the present invention to provide a low cost process for preparing an acid-stable alpha-amylase liquid concentrate.

The side activities sought to be avoided are amyloglucosidase and transferase.

The term transferase is employed to refer to a glucosyltransferase, which when acting on maltose gives rise to di- and tri-saccharides with other than 1.4-bonds, (e.g. isomaltose, panose or nigerose) and to free glucose.

Transferase activity presence therefore can be detected as a maltase activity. Such di- and tri-saccharides produced by transferase activity are non-fermentable, which property is most undesirable in both high dextrose, high maltose and high conversion syrups and accordingly, transferase products should be avoided in enzyme preparation for the production of such syrups.

The invention is based on the fact that the inventors have succeeded in mutating an alpha-amylase producing Aspergillus niger strain  to block its capability to produce amyloglucosidase and transferase, while retaining its ability to produce alpha-amylase.

The acid-stable alpha-amylase product according to the present invention may be used as liquefaction enzyme in the production of high dextrose and high maltose syrups or other starch hydrolysates. When using the alpha-amylase product in the liquefaction process, the same, low pH (about 4.3) employed in the saccharification process can be used making unnecessary a pH adjustment between the liquefaction and the saccharification steps. Furthermore, the alpha-amylase product is adequately thermostable to be used as a liquefaction enzyme.

Furthermore, an amylase free of amyloglucosidase activity is necessary in the production of high maltose syrups.

### SUMMARY OF THE INVENTION

According to a first aspect of the present invention there is provided an acid-stable alpha-amylase preparation suitable for high dextrose  high maltose and high conversion syrup production comprising an <u>Aspergillus</u> derived alpha-amylase liquid concentrate, characterized in that said alpha-amylase concentrate has neither amyloglucosidase nor transferase activities.

According to a further aspect of the present invention there is provided a process for preparing an acid-stable alpha-amylase concentrate with neither amyloglucosidase nor transferase activities, characterized in that an <u>Aspergillus</u> strain, mutated to block its capability to produce amyloglucosidase and transferase while retaining its ability to produce alpha-amylase, is cultivated in a nutrient medium containing assimilable sources of carbon and nitrogen and inorganic salts, followed by conversion of the alpha-amylase culture medium into the concentrate.

The enzyme recovery methods <u>per se</u> are well established in the art and usually comprise filtration, centrifugation or other clarification of the culture broth, followed by some evaporation of the liquid to generate the alpha-amylase concentrate.

Thus, the process of the present invention is a convenient and inexpensive way to prepare the acid-stable alpha-amylase preparation because the burdensome purification steps heretofore necessary to obtain a crystalline form alpha-amylase are avoided.

The present invention also provides mutant strains of Aspergillus essentially blocked as regards to synthesis of enzymes with amyloglucosidase and transferase activities, while retaining the ability to synthesize alpha-amylase.

According to a preferred embodiment of the present invention the amylase activity of the alpha-amylase preparation is in the range of from 50-1000 FAU per ml.

In a preferred embodiment of the present invention a strain of Aspergillus niger has been mutated to block the synthesis of enzymes with amyloglucosidase and transferase activities while retaining fully the ability to synthesize alpha-amylase. A culture of such a mutant strain of Aspergillus niger TSA-1 has been deposited with the German Collection of Microorganisms (DSM), Göttingen, Germany, on 2nd September, 1983 and has been assigned the reference number DSM 2761.

This strain can, of course, be mutated further or otherwise transformed, as, for example, for increased capability to produce the acid-stable alpha-amylase and further transformation of the strain is contemplated in practice of the invention.

It is known that Aspergillus strains cultivated in a glucose-containing medium generate sugar alcohols, vide for instance W.H. Lee, Can.J.Microbiol., 16, pp. 363 - 367, 1970. Such sugar alcohols accumulate in the culture broth and exert a stabilizing effect on enzyme solutions made from the culture broth, vide R.D. Schmid, Advances in Biochemical Engineering, Vol. 12, pp. 59 - 61, 1979. Cultivation of DSM 2761 generates sugar alcohols in the broth and the sugar alcohols carry through to the enzyme concentrate product.

Accordingly, a further aspect of the present invention is provision of an acid-stable alpha-amylase preparation with improved stability for containing therein culture broth derived sugar alcohols, preferably in an amount of at least above 1% by weight.

According to a further aspect the present invention provides a process for converting starch into high dextrose or high maltose syrups or other starch hydrolysates wherein the liquefaction or partial hydrolysis of starch is conducted in the presence of an enzyme system comprising an effective amount of the novel acid-stable α-amylase enzyme preparation.

The present invention also provides a process for converting starch into high maltose or high conversion syrups wherein the saccharification of starch or starch hydrolysates (preferably enzyme or acid liquefied starch) is conducted in the presence of an enzyme system containing an effective amount of the novel acid-stable α-amylase enzyme preparation. In the preparation of a high maltose syrup the saccharification is preferably conducted on a starch hydrolysate of 30 - 45 per cent by weight of dry solids and in a pH-range of from 3 to 5 and at a temperature in the range of from 50 to 75°C.

## DETAILED DESCRIPTION OF THE INVENTION

Mutation Procedure

Mutation of an amylase producing strain of Aspergillus niger to block its synthesis of amyloglucosidase and transferase was conducted by a succesive mutation procedure in the following manner.

Amyloglucosidase production was eliminated by γ-ray mutation. Conidia were harvested from an A. niger strain CBS 263 - 65 grown on a C-1 agar (3% sucrose, 0.1% $KH_2PO_4$, 0.3% $NaNO_3$, 0.05% $MgSO_4,7H_2O$, 0.01% $FeSO_4,7H_2O$, 0.05% KCl and 2.5% agar in demineralized water). The Conidia were filtered through a 28 μm nylon filter, collected by centrifugation, suspended in 10% skimmed milk and freeze-dried in glass ampules. The ampoules were irradiated with 125 Krad γ-ray from a Co source. The rate of survival was 1%.

Transferase activity production was eliminated by N-methyl-N'-nitro-N-nitrosoguanidin (NTG) mutation. Conidia were harvested, filtrated and centrifugated as described above and suspended in a salt solution (0.5 mM $KH_2PO_4$, 0.5 mM $K_2HPO_4$, 0.5 mM $(NH_4)_2HPO_4$, 0.05 mM $CaCl_2$, 0.05 mM $MgCl_2$, pH 7.2) and incubated under stirring at 34°C for 2 hours. NTG was then added (250 µg/ml) and the mutation was stopped 75 minutes later by washing the conidia several times in water.

## Selection of Mutants

Screening of the strains for absence of either amyloglucosidase or transferase activities may be carried out according to the same procedure: Conidia (from γ-ray treatment or NTG-treatment) were spread on YPG agar (yeast extract 4 g/liter, glucose 15 g/liter, $K_2HPO_4$ 1 g/liter, $MgSO_4$,$7H_2O$ 0.5 g/liter and agar 20 g/liter). After incubation at 34°C for 4 or 5 days agar slices each containing mycelium from a colony were cut out and arranged in hexagonal patterns on a glass plate and were then embedded in 1% agarose gel (pH 7.2, tris-buffer). In a hole, cut out centrally in each hexagon, the antiserum, against for instance amyloglucosidase, was placed.

The plates were incubated for 3 days at 4°C. Mutant colonies without precipitation lines were selected, purified by subcultivation and inoculated in shake flasks for analysis of the enzyme activities. Mutants without both amyloglucosidase and transferase activities can be isolated by successive use of this procedure, eliminating one enzyme activity at a time using monospecific antiserum against the individual enzymes.

## Determination of Enzyme Activity

One amylase unit (FAU) is defined as the amount of enzyme which under standard conditions (temperature 37°C, pH 4.7 (0.01 M acetate buffer) and reaction time 1 hour) hydrolyses 5.25 g starch (dry substance) (Merck) so that the hydrolyzed starch is only slightly coloured by addition of iodine- potassium iodide (4.04 g/liter KI and 0.022 g/liter $I_2$) and standardizing by comparing with an enzyme standard using a Daylite Comparator Illuminator. By this procedure both acid-stable as well as neutral (acid-unstable) alpha-amylase is measured.

Acid-stable alpha-amylase alone can be determined after inactivating of any neutral amylase if present by acid incubation with 0.1 M HCl at pH 2.5. After incubation at 37°C for 30 minutes pH is adjusted to 4.7 with NaOH. The acid-stable alpha-amylase is obtained in 100% yield by this procedure.

Preparation of the Enzyme

The present method of preparing an acid-stable alpha-amylase will now be described in more detail.

Spores of Aspergillus niger mutant TSA-1 are transferred from an agar slab to sterile water and transferred aseptically to an inoculation tank containing a suitable fermentation medium containing assimilable sources of carbon and nitrogen and inorganic salts. Stirring and aeration is started and fermentation is carried out at about 30 - 36°C until there is good growth in the tank (or for about 48 hours).

Part of the tank content is transferred to a main fermentation tank (2000 liter) containing a suitable fermentation medium containing assimilable carbon and nitrogen sources and inorganic salts. pH is about 5.0 - 6.5 and aerating and stirring is started. Fermentation is carried out at 30 - 36°C and pH is adjusted to about 3.2 - 5.0 after about 24 hours fermentation. The total fermentation time is about 100 - 200 hours.

The culture liquid is then freed from fungus mycelium by filtration and centrifugation. The broth liquid is concentrated, e.g. by evaporation and preserving agents may be added.

Typically, the resulting liquid enzyme concentrate exhibits an activity in the range of about 50 to 1000 FAU/ml.

**Enzyme Chemical Properties**

The temperature and pH dependence of the activity of the acid-stable alpha-amylase concentrate according to the present invention prepared as described hereinafter in Example 1 was determined by the method described above, using a reaction mixture of which the temperature and pH were adjusted to predetermined values. Reference is now made to the attached drawing in which

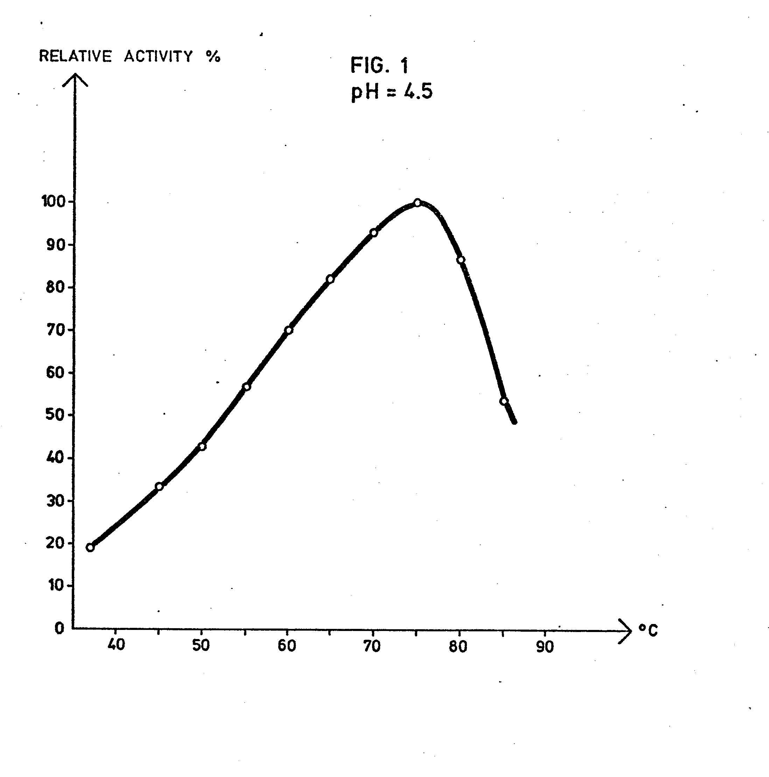

Fig. 1 graphically illustrates the relative activity plotted against temperature at pH 4.5, and

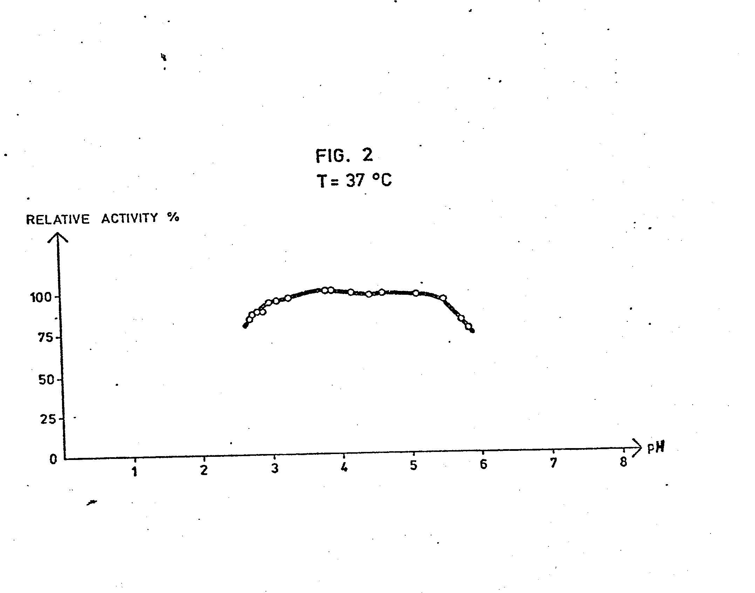

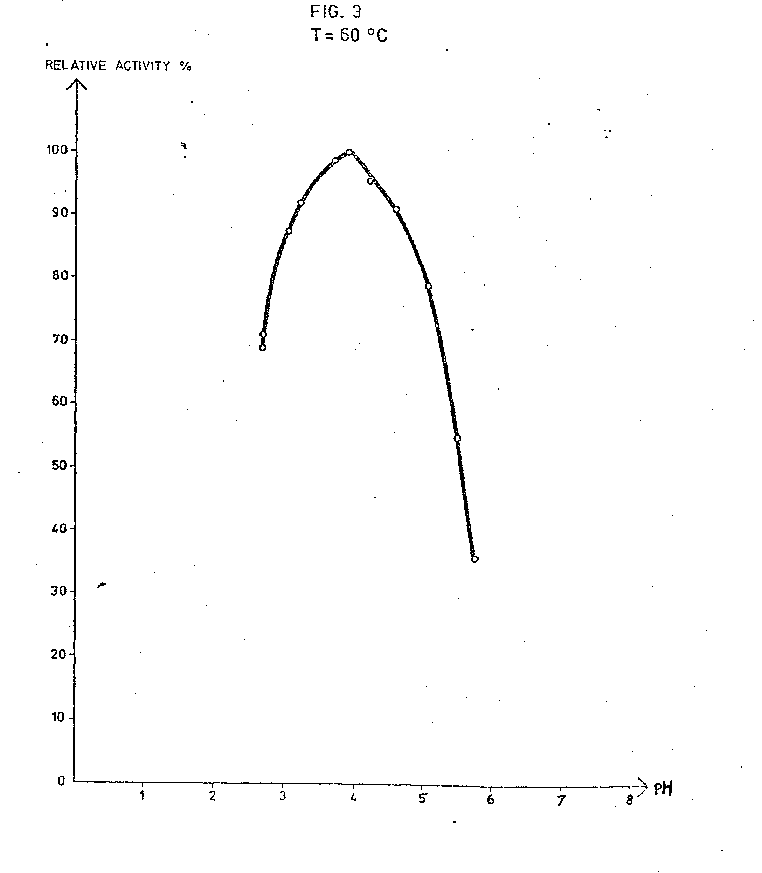

Fig. 2 and 3 graphically illustrate the relative activity plotted against pH (adjusted with acetate and citrate buffer) at 37° and 60°C, respectively.

The enzyme concentrate demonstrated an activity optimum of about 75°C at pH 4.5 and a pH optimum in the range of from 3 - 5.

The thermostability of the alpha-amylase according to the invention prepared as described in Example 1 was determined after addition of 100 mg of $Ca^{++}$ per liter concentrate and adjusting pH to 5.0. The residual amylase activity was determined by the method described above. The results appear in the following table:

Table I

| Temperature °C | Per cent residual activity after 30 min. |
|---|---|
| 70 | 100 |
| 75 | 82 |
| 80 | 10 |

The enzyme concentrate is without maltase activity (no glucose formation after 70 hours' incubation at 70°C of about 25% DS maltose with 0.32 FAU of enzyme per g maltose).

Practice of the invention is described in further detail by the following examples.

## Example 1

In an inoculation tank a volume of 300 liter of the following medium was prepared:

| | |
|---|---|
| Corn steep liquor: | 7.2 kg |
| Glucose: | 7.2 kg |
| $CaCO_3$: | 0.9 kg |
| Pluronic L 61: | 30 ml |
| Water: | up till 300 liter |

pH was adjusted to 5.5 before addition of $CaCO_3$ and sterilization. The mixture was inoculated with spores of strain TSA-1 from a Fernbach flask containing C-1 agar which had been incubated for about 7 days at 30°C. Stirring (300 rpm) and aeration (300 Nl/min) was started and fermentation was carried out at 34°C for about 51 hours.

150 liter of the tank content were then transferred to a main fermentation tank containing:

| | |
|---|---|
| Glucose: | 340 kg |
| Soy bean meal: | 85 kg |
| $KH_2PO_4$: | 5.1 kg |
| $Na_2HPO_4,12H_2O$ : | 6.8 kg |
| Pluronic L61: | 150 ml |
| Water: | to 1700 liter |

During the first 18 hours of fermentation aeration was slowly increased from 500 to 1700 Nl/min and stirring was increased from 0 to 250 rpm. The fermentation was carried out at 34°C. pH was 6.2 before inoculation and after fermentation for 24 hours was lowered slowly to 3.2 - 3.5 by addition of phosphorous acid. pH was kept at 3.2 - 3.5 during the rest of the fermentation. Total fermentation time was 140 - 160 hours.

The culture liquid containing 1.3 FAU/ml was pretreated with 3% Clarit BW 100 (Bentonit) at pH 4.0 and drum filtrated on a precoat vacuum drum filter with HSC as precoat (HSC kieselguhr from John Manville). The drum filtrate was filtrated on Seitz plates and then ultra/diafiltrated on Romicon PM-10 membranes.

After ultrafiltration the concentrate was filtrated on a frame filter press (Schule) with HSC and CBL/3 (kieselguhr from CECA) as filter aid. Finally the filtrate was evaporated until 38% RD (refractometer dry solid) and preserved with sodium benzoate (0.1%) and potassium sorbate (0.1%).

The so obtained enzyme concentrate had an enzyme activity of 67 FAU/ml. The content of sugar alcohols was 1.3% by weight; the concentrate exhibited no maltase activity.

After incubation for 0.5 hour at pH 2.5 and 37°C a residual amylase activity of 100% was found, indicating that no neutral alpha-amylase was present at the end of the fermentation process.

Example 2

Saccharification test with acid-stable alpha-amylase

A 7 DE maltodextrin was prepared by liquefying corn starch with Termamyl® according to standard procedure and then spray-drying.

Substrates for saccharification were then prepared from this material by redissolving suitable amounts in deionized water and adjusting the dry substance (DS) to 30%, 35% and 40% (w/w).

The pH was adjusted to 4.5 and an amount of enzyme corresponding to 0.16 FAU/g DS was added. The saccharification was carried out in standard laboratory reactors under constant stirring, at 70°C. The results are tabulated below:

## Table II

DS = 30%

| Reaction time hours | %DP$_1$ | %DP$_2$ | %DP$_3$ | %DP$_{4+}$ | pH |
|---|---|---|---|---|---|
| 2 | 0.7 | 14.0 | 23.0 | 62.3 | |
| 4 | 1.6 | 28.0 | 44.9 | 25.5 | |
| 22 | 6.1 | 48.9 | 25.4 | 19.6 | 4.13 |
| 48 | 9.1 | 52.8 | 19.9 | 18.2 | 4.18 |
| 72 | 12.0 | 54.4 | 16.2 | 17.4 | 3.78 |
| 96 | 13.8 | 55.3 | 13.9 | 17.0 | 3.80 |

DS = 35%

| | %DP$_1$ | %DP$_2$ | %DP$_3$ | %DP$_{4+}$ | pH |
|---|---|---|---|---|---|
| 2 | 0.6 | 13.3 | 21.0 | 65.1 | |
| 4 | 1.5 | 25.3 | 45.4 | 27.8 | |
| 22 | 6.3 | 48.7 | 25.1 | 19.8 | 4.14 |
| 48 | 9.2 | 51.5 | 19.9 | 19.4 | 4.06 |
| 72 | 13.6 | 52.8 | 15.4 | 17.8 | 3.74 |
| 96 | 15.4 | 54.6 | 13.1 | 17.0 | 3.74 |

DS = 40%

| | %DP$_1$ | %DP$_2$ | %DP$_3$ | %DP$_{4+}$ | pH |
|---|---|---|---|---|---|
| 2 | 0.6 | 12.1 | 19.1 | 68.1 | |
| 4 | 1.4 | 23.4 | 44.5 | 30.7 | |
| 22 | 7.0 | 48.5 | 24.7 | 19.8 | 4.08 |
| 48 | 10.6 | 50.6 | 19.4 | 18.9 | 4.05 |
| 72 | 14.6 | 52.6 | 15.3 | 17.5 | 3.71 |
| 96 | 17.4 | 53.0 | 12.9 | 16.7 | 3.73 |

From these results it can be seen that maltose (DP$_2$) is the main product of hydrolysis and that the amount

of fermentable sugars ($DP_1$ + $DP_2$ + $DP_3$) is high. The glucose content ($DP_1$ increases with increasing DS.

Example 3

Use of the acid-stable α-amylase as a post-liquefaction enzyme

The acid-stable α-amylase was tested as a post-liquefaction enzyme for high maltose syrup production where a low initial DE is required.

A 32% DS starch slurry was liquefied with Termamyl® under the following conditions:

Starch slurry: Corn starch, 32% DS
Calcium:        70 ppm
pH:             6.0 - 6.2
Termamyl®120L:  1 kg/ton DS or 0.12 KNU/g DS
Jet cooker:     110 - 112°C
Flow:           350 l/h
Holding time:   5 minutes

The liquefied starch was flash-cooled to 90°C and collected in an intermediate holding tank where the pH was adjusted to 5.2. The acid-stable α-amylase was continuously metered into the holding tank at a rate which gave a dosage corresponding to       0.08 FAU/g DS.

The liquefied starch was then pumped through a Mixco converter where  the residence time was 20 - 25 minutes.

After liquefaction, the DE value was found to be 3.0.

The liquefied starch substrate was cooled to $60^{o}$C and collected in a saccharification tank.

0.4 PUN/g DS pullulanase (PROMOZYME ® 200L) and 0.045 $\beta$U/g DS barley $\beta$-amylase were added to each tank at the start of saccharification.

The filterability of the saccharified syrup was measured after 24 hours using the standard CST method. The carbohydrate composition was measured by HPLC after 20 and 48 hours. An iodine test was performed after 20 hours. The following results were obtained.

| Time,h | %DP$_1$ | %DP$_2$ | %DP$_3$ | %DP$_{4+}$ |
|---|---|---|---|---|
| 20 | 0.5 | 70.7 | 17.1 | 11.7 |
| 48 | 0.8 | 71.9 | 18.5 | 8.8 |
| Control* 48 | 0.2 | 70.7 | 5.7 | 23.4 |

*  3.5 DE Termamyl ®maltodextrin

The final syrup showed a good filterability and was starch-free whereas the control gave a starch positive reaction and a non-filterable syrup.

The final syrup contained more than 91% fermentable sugars whereas the control syrup produced without the acid-stable $\alpha$-amylase only contained about 77% fermentable sugars.

Accordingly, the acid-stable $\alpha$-amylase of the present invention is well suited as a post-liquefaction enzyme giving low DE maltodextrins which during a subsequent saccharification step can be converted into starch-free syrups with a high content of fermentable sugar. Such syrups are not only ideal syrups for use in the confectionary industry, but also good substrates for further saccharification to potentially higher maltose levels.


## Example 4

### Use of the acid-stable α-amylase as a liquefaction enzyme in the alcohol industry

The pH profile of the acid-stable α-amylase makes it better suited for the natural pH of distillery mashes than the traditional bacterial α-amylases. The liquefaction trials were carried out in 400 ml beakers furnished with an electrical heating coil and an FL 10 spindle (Haake Viscosimeter VT 180) as agitator. A 20% slurry (15% starch) of corn grits was made in deionised water (70g corn grits + 280 g water). $CaCl_2$, $2H_2O$ (50 mg $Ca^{++}$/litre) and α-amylase (70.5 FAU/g) were added. After adjusting the pH to 4.0 - 5.0, the temperature was raised to 75°C (1°C/min) and held for an hour. The viscosity was measured frequently during the heating and holding period and increased from approximately 200 cP to 500 - 700 cP when the temperature was raised to 75°C, but dropped to approximately 300 cP when the temperature was maintained at 75°C, due to the liquefying properties of the acid-stable amylase. (The viscosity was measured on a Haake Viscosimeter VT 180 with a FL 10 spindle). The viscosity pattern shows a reasonably good liquefaction action of the novel α-amylase for use in the alcohol industry.

The features disclosed in the foregoing description, in the following claims and/or in the accompanying drawings may, both separately and in any combination thereof, be material for realising the invention in diverse forms thereof.

CLAIMS

1. An acid-stable alpha-amylase preparation comprising an Aspergillus derived alpha-amylase concentrate, wherein the concentrate was prepared from a culture broth wherein the alpha-amylase was generated free of amyloglucosidase and transferase activities on cultivation.

2. A preparation according to claim 1, wherein the concentrate has been prepared from an alpha-amylase producing Aspergillus strain mutated to block its capability to produce amyloglucosidase and transferase activities, whilst retaining its ability to produce alpha-amylase.

3. A preparation according to claim 2, wherein the mutated Aspergillus strain is a mutated Aspergillus niger strain.

4. A preparation according to claim 3, wherein the mutated Aspergillus niger strain is the strain deposited under number DSM 2761.

5. A preparation according to any of the preceding claims, wherein the concentrate comprises at least 1% by weight of culture broth derived sugar alcohols.

6. A preparation according to any of the preceding claims, wherein the concentrate has an alpha-amylase activity in the range of from 50 - 1000 FAU/ml.

7. A process for preparing an acid-stable alpha-amylase liquid concentrate free of amyloglucosidase and transferase activities, which process comprises cultivating an alpha-amylase producing Aspergillus strain, mutated to block its capability to produce amyloglucosidase and transferase activities whilst retaining its ability to produce alpha-amylase in a nutrient medium containing assimilable sources of carbon and nitrogen and inorganic salts and thereafter recovering and concentrating the alpha-amylase culture medium.

8. A process according to claim 7 wherein the Aspergillus strain is an Aspergillus niger strain.

9. A process according to claim 8, wherein the Aspergillus niger strain is strain DSM 2761 or mutants or variations thereof.

10. An Aspergillus strain, mutated to essentially block its capability of synthesizing amyloglucosidase and transferase activities whilst retaining the ability to synthesize a-amylase.

11. An Aspergillus strain according to claim 10 being DSM 2761 or mutants or variations thereof.

12. A process for converting starch into high dextrose or high maltose syrups or other starch hydrolysates, which process comprises conducting the liquefaction or partial hydrolysis of starch in the presence of an enzyme system which comprises an effective amount of the acid-stable alpha-amylase concentrate according to claims 1 - 6.

13. A process for converting starch into high maltose or high conversion syrups, which process comprises conducting the saccharification of starch or starch hydrolysates in the presence of an enzyme system containing an effective amount of the acid-stable alpha-amylase concentrate according to claims 1 - 6 and optionally a saccharifying enzyme selected from the group consisting of glucoamylase and beta-amylase and/or a debranching enzyme.

14. A process according to claim 13 wherein the starch hydrolysates are either enzyme or acid liquefied starch.

15. A process according to claim 13 for converting starch into high maltose syrups further comprising saccharification of a starch hydrolysate of 30 to 45 per cent by weight of dry solids.

16. A process according to claim 15 wherein the saccharification step is conducted in a pH-range of from 3 to 5 and at a temperature in the range of from 50 to 75°C.

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits: DSM 2761

FIG. 1
pH = 4.5

FIG. 2
T= 37 °C

RELATIVE ACTIVITY %

0138428

3/3

FIG. 3
T = 60 °C